# EUROPEAN PATENT SPECIFICATION

(11) **EP 2 547 276 B1**
(45) Date of publication and mention of the grant of the patent: **31.08.2016**
(21) Application number: 11709271.8
(22) Date of filing: 08.03.2011
(51) Int. Cl.: A61B 18/14

(54) **ABLATION CATHETER WITH ISOLATED TEMPERATURE SENSING TIP**
ABLATIONSKATHETER MIT ISOLIERTER TEMPERATURMESSSPITZE
CATHÉTER D'ABLATION À POINTE ISOLÉE POUR DÉTECTER LA TEMPÉRATURE

(30) Priority: 15.03.2010 US 313936 P
(43) Date of publication of application: 23.01.2013
(73) Proprietor: Boston Scientific Scimed, Inc., Maple Grove, MN 55311-1566 (US)
(72) Inventor: KOBLISH, Josef, Sunnyvale, CA 94087 (US); BENCINI, Robert, F., Sunnyvale, CA 94086 (US); KIM, Isaac, San Jose, CA 95135 (US); FORREST, Mark, Sunnyvale, CA 94085 (US); CHEN, Patricia, Fremont, CA 94538 (US); RANKIN, Darrell, L., Milpitas, CA 95035 (US); TEE, Siew-Hung, San Jose, CA 95110 (US)
(74) Representative: Pfenning, Meinig & Partner mbB
(86) International application number: PCT/US2011/027591
(87) International publication number: WO 2011/115787

(56) References cited:
- US-A- 6 053 912
- US-A1- 2007 043 397
- US-A1- 2008 091 193
- US-A1- 2008 161 797

## Description

### TECHNICAL FIELD

This application relates generally to medical devices and, more particularly, to systems and methods related to temperature sensing and ablation catheters.

### BACKGROUND

Aberrant conductive pathways disrupt the normal path of the heart's electrical impulses. For example, conduction blocks can cause the electrical impulse to degenerate into several circular wavelets that disrupt the normal activation of the atria or ventricles. The aberrant conductive pathways create abnormal, irregular, and sometimes life-threatening heart rhythms called arrhythmias. Ablation is one way of treating arrhythmias and restoring normal contraction. The sources of the aberrant pathways (called focal arrhythmia substrates) are located or mapped using mapping electrodes. After mapping, the physician may ablate the aberrant tissue. In radio frequency (RF) ablation, RF energy is directed from the ablation electrode through tissue to ablate the tissue and form a lesion.

Simple RF ablation catheters have a small tip and therefore most of the RF power is dissipated in the tissue. The advantage is that the lesion size is somewhat predictable from the RF power and time. However, the tissue can get very hot at the contact point, and thus there can be a problem of coagulum formation.

Various designs have been proposed to cool the ablation electrode and surrounding tissue to reduce the likelihood of a thrombus (blood clot), prevent or reduce impedance rise of tissue in contact with the electrode tip, and increase energy transfer to the tissue because of the lower tissue impedance. Catheters have been designed with a long tip for contact with blood to provide convective cooling through blood flow, which reduces the maximum temperature at the contact point. However, the amount of cooling depends on local blood velocity, which is uncontrolled and is generally not known. Since the convective heat transfer coefficient depends on the blood velocity, the tip temperature varies with blood velocity even at constant conduction power from tissue to tip. Thus, the electrophysiologist is less able to predict the lesion size and depth, as the amount of power delivered into the tissue is not known. Closed irrigation catheters provide additional cooling to the tip, which keeps the tissue at the contact point cooler with less dependence on the local blood velocity. However, the added cooling further masks the amount of RF ablation power dissipated into the tissue. The tip temperature is poorly correlated to the tissue temperature. Open irrigation catheters cover the tissue near the tip with a cloud of cool liquid to prevent coagulum in the entire region. However, more cooling fluid is used, which further masks the amount of RF power that enters the tissue.

If the amount of power entering the tissue is masked, then the size of the lesion cannot be accurately predicted. The RF power entering the tissue and the temperature profile versus time in the tissue is highly uncertain, which may contribute to under treatment or over treatment. If too much power is used, the tissue temperature may rise above 100° C and result in a steam pop. Steam pops may tear tissue and expel the contents causing risk of embolic damage to the circulation. Additionally, the temperature differs throughout a volume of tissue to be ablated. A steam pop may occur in one part of the tissue volume before the tissue in other parts of the tissue volume reaches a temperature over 50° C and is killed. As a consequence, power may be cautiously applied to avoid steam pop, and the tissue may be under treated resulting in the lesion being smaller than desired. The result of under treatment may be failure to isolate the tissue acutely or chronically, resulting in an inadequate clinical treatment of the arrhythmia.

US 6,053,912 relates to systems and methods for sensing sub-surface temperatures in body tissue during ablation with actively cooled electrodes. The electrode shown therein is adapted to be connected to a source of ablation energy to conduct ablation energy for transmission by the electrode into tissue at the tissue-electrode interface. The systems and methods disclosed therein also include an element to cool the electrode. The systems and methods shown therein hold a tissue temperature sensing element in a carrier in thermal conductive contact with tissue beneath the tissue electrode interface. In Fig. 13, US 6,053,912 discloses an ablation catheter including a catheter body, a thermocouple component 112, and a sleeve 136 made of thermally insulating material. This sleeve extends from the distal tip through the electrode and into the catheter body.

### SUMMARY

Disclosed herein, among other things, are methods and apparatus related to radio frequency (RF) ablation catheters. The present subject matter provides an ablation catheter system including a catheter body with a distal tip, and a thermocouple component at the distal tip. According to an embodiment, the thermocouple component protrudes from the distal tip. The thermocouple component is adapted to sense temperature of bodily fluid and/or tissue. The system includes a non-conductive insert configured to physically separate and thermally insulate the thermocouple component from the catheter body. According to an embodiment, the non-conductive insert includes a ceramic material.

According to various embodiments, the system includes an open-irrigated ablation catheter system. The open-irrigated system includes a catheter body with a distal tip and at least one fluid chamber. The system also includes a plurality of irrigation ports within the catheter body, where the plurality of irrigation ports enable fluid to exit from the at least one fluid chamber. A thermocouple component at the distal tip is adapted to sense temperature of bodily fluid and/or tissue. The system further includes a non-conductive insert configured to physically separate and thermally insulate the thermocouple component from the catheter body and the plurality of irrigation ports.

This Summary is an overview of some of the teachings of the present application and not intended to be an exclusive or exhaustive treatment of the present subject matter. Further details about the present subject matter are found in the detailed description and appended claims. The scope of the present invention is defined by the appended claims and their equivalents.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG. 1A-1B illustrate planar and cross-sectional views of an ablation catheter system, according to an embodiment of the present subject matter.
FIG. 2A-2B illustrate planar and cross-sectional views of an ablation catheter system with multiple thermocouple components, according to an embodiment of the present subject matter.
FIG. 3A-3B illustrate planar and cross-sectional views of an open-irrigated ablation catheter system, according to an embodiment of the present subject matter.
FIG. 4A-4B illustrate planar and cross-sectional views of an open-irrigated ablation catheter system with multiple thermocouple components, according to an embodiment of the present subject matter.

### DETAILED DESCRIPTION

The following detailed description refers to subject matter in the accompanying drawings which show, by way of illustration, specific aspects and embodiments in which the present subject matter may be practiced. These embodiments are described in sufficient detail to enable those skilled in the art to practice the present subject matter. References to "an," "one," or "various" embodiments in this disclosure are not necessarily to the same embodiment, and such references contemplate more than one embodiment. The following detailed description is, therefore, not to be taken in a limiting sense, and the scope is defined only by the appended claims, along with the full scope of legal equivalents to which such claims are entitled.

During an RF ablation procedure, high RF current density near the electrode causes resistive heating in the tissue. This heat is also transferred by convection to surrounding tissue. RF electric current is applied to tissue to locally heat a region of the tissue to a temperature that kills cells (e.g. over 50° C throughout the volume of tissue to be ablated). However, undesired steam pops may occur if the temperature of a portion of the tissue rises to or above 100° C. Therefore, the temperature of the tissue to be ablated should be above 50° C throughout the volume but should not reach 100 ° C anywhere in the volume.

Irrigated ablation catheters typically have poor temperature sensing capabilities because the cooling flow runs directly over the sensor and creates an artificially low temperature at the sensor. The present subject matter minimizes the effects of the cooling flow by thermally insulating the sensor from the fluid.

The present subject matter relates to a RF ablation catheter that includes an isolated temperature sensing tip. The temperature sensing feature is valuable for preventing excessive heating of the ablation electrode and surrounding tissue. In addition, accurate temperature readings of target tissue at an ablation site are important for applying proper intensity and duration of ablation. By providing a more accurate real time temperature of the electrode tip, coagulum formation on the catheter tip can be reduced. A reduction in steam pops caused by high temperatures is also possible using the present subject matter.

To more accurately measure temperature of an ablation catheter and surrounding tissue, a temperature sensing component or thermocouple (TC) is positioned in the center of the tip of the catheter, perpendicular to the tip surface, and is adapted for tissue contact in various embodiments. The temperature sensing component can be positioned in other orientations besides perpendicularly to the tip surface, in various embodiments. The temperature sensing component includes a thermistor, in an embodiment. The temperature sensing component can be formed from a metal or metals, or from a ceramic or polymer material, in various embodiments. Other types of temperature sensing components can be used without departing from the scope of this disclosure. The temperature sensing component protrudes from the tip surface, in an embodiment. In other embodiments, the temperature sensing component is recessed from or flush with the tip surface. The temperature sensing component is surrounded by a non-conductive insert. The non-conductive insert includes a highly porous ceramic material, or ceramic insert, in various embodiments. The non-conductive insert is positioned such that it insulates and isolates the temperature sensing component from resistive heating after RF energy is delivered from the tip to perform ablation.

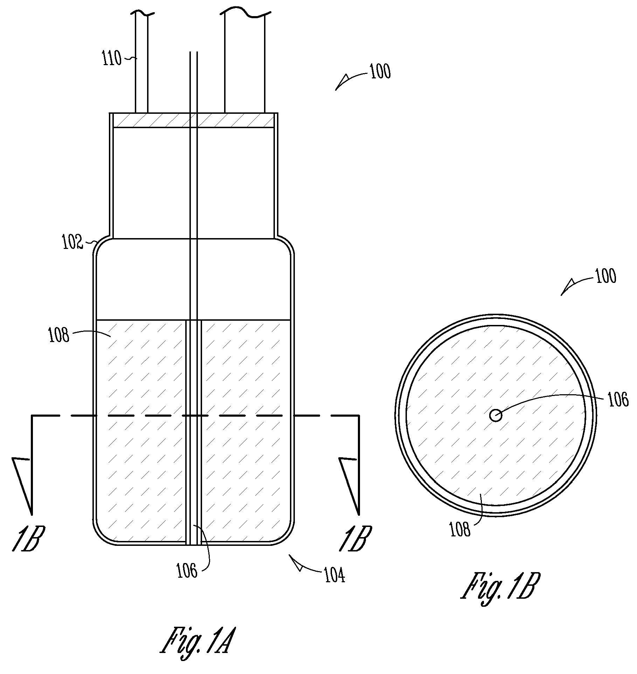

FIG. 1A-1B illustrate planar and cross-sectional views of an ablation catheter system, according to an embodiment of the present subject matter. The ablation catheter system 100 includes a catheter body 102 with a distal tip 104, and a thermocouple component 106 at the distal tip. The thermocouple component 106 is adapted to sense temperature of bodily fluid and/or tissue. The thermocouple component protrudes from the tip surface, in an embodiment. In other embodiments, the thermocouple component is recessed from or flush with the tip surface. The system 100 includes a non-conductive insert 108 configured to physically separate and thermally insulate the thermocouple component 106 from the catheter body 102. The non-conductive insert includes a highly porous ceramic material, or ceramic insert, in various embodiments. The catheter system includes a center support 110 within the catheter body, in various embodiments.

According to various embodiments, the non-conductive insert is further configured to electrically isolate the thermocouple component from the catheter body. The catheter body further includes proximal ring electrodes adapted for ECG mapping, in various embodiments, and the non-conductive insert is further adapted to electrically isolate the thermocouple component from the proximal ring electrodes. The non-conductive insert includes a porous ceramic material including a large air void percentage per volume, in an embodiment. In various embodiments, the non-conductive insert includes any insulating material with a low coefficient of thermal transfer. The non-conductive insert can be made from any material that would provide thermal and/or electrical isolation of the thermocouple or thermocouples from the tip or body. In various embodiments, the non-conductive insert includes any ceramic (porous or non-porous), polymeric, adhesive, epoxy, or any other thermal or electrically non-conductive material. In one embodiment, the non-conductive insert is cylindrically shaped. In other embodiments, the non-conductive insert can be any shape or configuration to isolate one or more thermocouples and/or one or more cooling fluid flow paths. In various embodiments, the non-conductive insert can be porous, slotted/scalloped external profile or have discrete internal fluid passageways such as holes or slots.

The thermocouple component is adapted to protrude from a center of the distal tip, in an embodiment. In other embodiments, the thermocouple component is contained within the tip. The thermocouple component is flush with the tip, in further embodiments. In various embodiments, multiple secondary or alternate thermocouples are arranged in a predetermined configuration (geometrically symmetrical, non-symmetrical patterns, radially or axially positioned, linear, randomly located, or other configuration) around or along the tip. Other thermocouple configurations are possible without departing from the scope of this disclosure. In various embodiments, the distal tip has a circular cross section. Other geometries are possible without departing from the scope of this disclosure. The thermocouple component is adapted to protrude approximately 1 mm from the distal tip, in one embodiment. The thermocouple component is adapted to protrude approximately 0.5 mm to 1.5 mm from the distal tip, in various embodiments. The thermocouple component is adapted to protrude greater than approximately 1 mm from the distal tip, in another embodiment. The system further includes components common to a bi-directional steerable ablation catheter, such as a center support, steering pull wires, mapping electrode wires and radio frequency wires in various embodiments. The thermocouple is positioned perpendicular to a side wall of the distal tip in the depicted embodiment, but other orientations of the thermocouple are possible without departing from the scope of this disclosure.

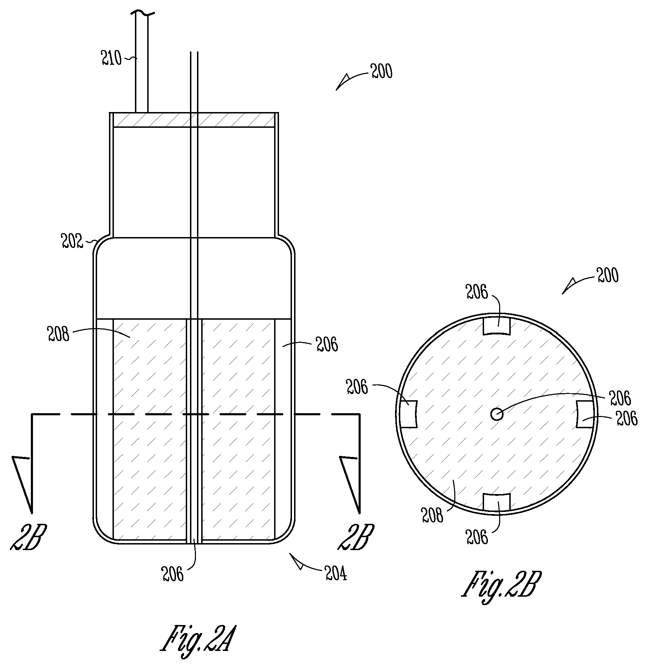

FIG. 2A-2B illustrate planar and cross-sectional views of an ablation catheter system with multiple thermocouple components, according to an embodiment of the present subject matter. The ablation catheter system 200 includes a catheter body 202 with a distal tip 204, and thermocouple components 206. The thermocouple components 206 are adapted to sense temperature of bodily fluid and/or tissue. The system 200 includes a non-conductive insert 208 adapted to physically separate and thermally insulate the thermocouple components 206 from the catheter body 202. The catheter system includes a center support 210 within the catheter body, in various embodiments. The depicted embodiment includes four additional thermocouples 206 positioned 90 degrees apart. Alternate orientations of the thermocouples, which are positioned perpendicular to the tip side wall, are possible without departing from the scope of this disclosure. According to various embodiments, the catheter system can include n thermocouples positioned 360/n degrees apart in addition to or in place of a centered thermocouple. Other arrangements of the thermocouples are possible without departing from the scope of this disclosure. Each of the thermocouples is encased by ceramic insulating material, in various embodiments.

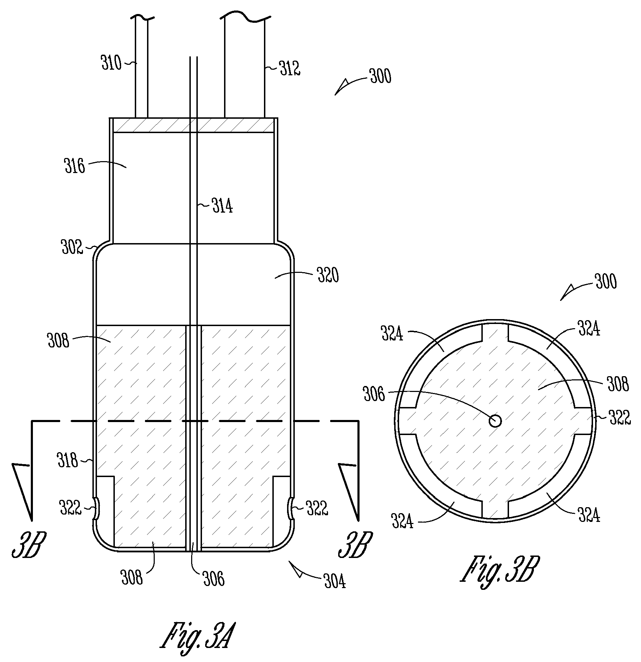

FIG. 3A-3B illustrate planar and cross-sectional views of an open-irrigated ablation catheter system, according to an embodiment of the present subject matter. The open-irrigated system 300 includes a catheter body 302 with a distal tip 304 and at least one fluid chamber 320. The system 300 also includes a plurality of irrigation ports 322 within the catheter body, where the plurality of irrigation ports 322 enable fluid to exit from the at least one fluid chamber 320 via a plurality of fluid flow channels 324. A thermocouple component 306 at the distal tip is adapted to sense temperature of bodily fluid and/or tissue. The system further includes a non-conductive insert 308 adapted to physically separate and thermally insulate the thermocouple component 306 from the catheter body 302, the plurality of irrigation ports 322 and the plurality of fluid flow channels 324. In various embodiments, the system includes a center support 310, cooling lumens 312, thermocouple wires 314, a proximal insert 316, and one or more electrodes 318. The system also includes components common to a bi-directional steerable ablation catheter, such as steering pull wires and radio frequency wires in various embodiments. The thermocouple wires attach to a connector at the back of the catheter that is attached to an RF generator having a temperature control algorithm, in various embodiments.

The thermocouple is surrounded by a highly porous ceramic material, and the ceramic material is positioned such that it insulates and isolates the temperature sensing component from the turbulent fluid in the proximal cooling chamber designed to cool the tip, in various embodiments. The fluid flow channels are oriented along the length of the cathode body, separated from the temperature sensing component by the ceramic material. Four or more fluid flow channels are used, in various embodiments. Cooling lumens that run the length of the catheter shaft supply the irrigation fluid, in an embodiment.

A cooling fluid, such as a saline, is delivered through the catheter to the catheter tip, where the fluid exits through irrigation ports to cool the electrode and surrounding tissue. Clinical benefits of such a catheter include, but are not limited to, controlling the temperature and reducing coagulum formation on the tip of the catheter, preventing impedance rise of tissue in contact with the catheter tip, and maximizing potential energy transfer to the tissue.

According to various embodiments, the non-conductive insert is further adapted to electrically isolate the thermocouple component from the catheter body, and/or to insulate the thermocouple component from the proximal cooling chamber. The fluid chamber includes a proximal cooling chamber, in an embodiment. The thermocouple is connected via wires to a processor adapted to calculate an amount of fluid needed at the distal tip to cool surrounding tissue based on sensed temperature, in various embodiments. According to various embodiments, the fluid flow channels are oriented such that fluid passing through the catheter and out the distal tip passes along side of an outside diameter of the ceramic material. Thus, the proximal cooling fluid is isolated from the thermocouple by the non-conductive insert. The catheter system combines an open irrigation configuration with accurate temperature sensing capability at the tip of the catheter, which helps prevent excessive heating of the ablation electrode.

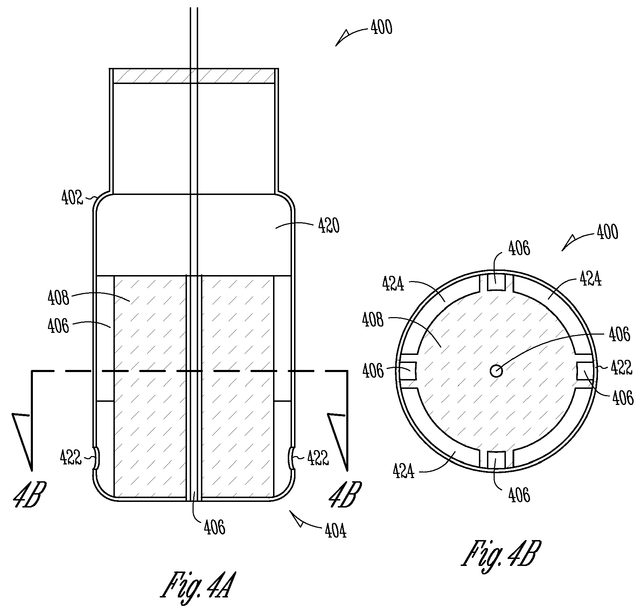

FIG. 4A-4B illustrate planar and cross-sectional views of an open-irrigated ablation catheter system with multiple thermocouple components, according to an embodiment of the present subject matter. The open-irrigated system 400 includes a catheter body 402 with a distal tip 404 and at least one fluid chamber 420. The system 400 also includes a plurality of irrigation ports 422 within the catheter body, where the plurality of irrigation ports 422 enable fluid to exit from the at least one fluid chamber 420 via a plurality of fluid flow channels 424. Thermocouple components 406 at the distal tip are adapted to sense temperature of bodily fluid and/or tissue. The system further includes a non-conductive insert (such as a ceramic material) 408 configured to physically separate and thermally insulate the thermocouple components 406 from the catheter body 402, the plurality of irrigation ports 422 and the plurality of fluid flow channels 424. The depicted embodiment includes four additional thermocouples 406 positioned 90 degrees apart. According to various embodiments, the catheter system can include n thermocouples positioned 360/n degrees apart in addition to or in place of a centered thermocouple. Other arrangements of the thermocouples are possible without departing from the scope of this disclosure, including those that are not equally spaced around the circumference of the tip.

One of ordinary skill in the art will understand that, the modules and other circuitry shown and described herein can be implemented using software, hardware, and/or firmware. Various disclosed methods may be implemented as a set of instructions contained on a computer-accessible medium capable of directing a processor to perform the respective method.

## Claims

1. An ablation catheter system, comprising:
a catheter body (302; 402) including a distal tip (304; 404);
a thermocouple component (306; 406) at the distal tip, wherein the thermocouple component is adapted to sense temperature of bodily fluid and/or tissue;
a non-conductive insert (308; 408) configured to physically separate and thermally insulate the thermocouple component (306; 406) from the catheter body (302; 402); and
wherein the catheter body further includes at least one fluid chamber (320; 420), and the system further comprises:
a plurality of irrigation ports (322; 422) within the catheter body (302; 402), wherein the plurality of irrigation ports (322; 422) enable fluid to exit from the at least one fluid chamber (320; 420) via a plurality of fluid flow channels (324; 424), said channels being separated from the thermocouple component by the non-conductive insert (308;408), wherein the non-conductive insert (308; 408) is configured to physically separate and thermally insulate the thermocouple component from the plurality of irrigation ports (322; 422); and
wherein the fluid chamber includes a proximal cooling chamber situated proximally of the non-conductive insert (308; 408).

2. The system of claim 1, wherein the thermocouple component (306; 406) is contained within the distal tip.

3. The system of claim 1, wherein the thermocouple component (306; 406) is flush with the distal tip.

4. The system of claim 1, wherein the thermocouple component (306; 406) protrudes from the distal tip.

5. The system of any of the preceding claims, wherein the non-conductive insert (308; 408) includes a ceramic material.

6. The system of any of the preceding claims, wherein the non-conductive insert (308; 408) is further adapted to electrically isolate the thermocouple component (306; 406) from the catheter body (302; 402).

7. The system of claim 4, wherein the thermocouple component (306; 406) is adapted to protrude from a center of the distal tip (304; 404).

8. The system of any of the preceding claims, wherein the distal tip (304; 404) has a circular cross section.

9. The system of claim 4, wherein the thermocouple component is adapted to protrude approximately 1 mm from the distal tip (304; 404).

10. The system of claim 4, wherein thermocouple component is adapted to protrude approximately 0.5 mm to 1.5 mm from the distal tip (304; 404).

11. The system of any of the preceding claims, wherein the thermocouple is positioned perpendicular to a side wall of the distal tip (304; 404).

12. The system of claim 11, further comprising four thermocouples positioned 90 degrees apart.

13. The system of claim 1, wherein the thermocouple is connected to a processor adapted to calculate an amount of fluid needed at the distal tip (304; 404) to cool surrounding tissue based on sensed temperature.

14. The system of claim 1 or claim 13, wherein the irrigation ports (322; 422) are oriented such that fluid passing through the catheter and out the distal tip (304; 404) passes along side of an outside diameter of the non-conductive insert (308; 408).

## Patentansprüche

1. Ablationskathetersystem, welches aufweist:
einen Katheterkörper (302; 402) enthaltend eine distale Spitze (304; 404);
eine Thermoelementkomponente (306; 406) an der distalen Spitze, wobei die Thermoelementkomponente ausgestaltet ist zum Erfassen der Temperatur von Körperfluid und/oder Gewebe,
einen nichtleitenden Einsatz (308; 408), der konfiguriert ist zum physischen Trennen und thermischen Isolieren der Thermoelementkomponente (306; 406) von dem Katheterkörper (302; 402); und
wobei der Katheterkörper weiterhin zumindest eine Fluidkammer (320; 420) enthält und das System weiterhin aufweist:
mehrere Benetzungsöffnungen (322; 422) innerhalb des Katheterkörpers (302; 402), wobei die mehreren Benetzungsöffnungen (322; 422) ermöglichen, dass Fluid aus der zumindest einen Fluidkammer (320; 420) über mehrere Fluidströmungskanäle (324; 424) austritt, wobei diese Kanäle durch den nichtleitenden Einsatz (308; 408) von der Thermoelementkomponente getrennt sind,
wobei der nichtleitende Einsatz (308; 408) konfiguriert ist zum physischen Trennen und thermischen Isolieren der Thermoelementkomponente von den mehreren Benetzungsöffnungen (322; 422); und
wobei die Fluidkammer eine proximale Kühlkammer enthält, die proximal von dem nichtleitenden Einsatz (308; 408) angeordnet ist.

2. System nach Anspruch 1, bei dem die Thermoelementkomponente (306; 406) innerhalb der distalen Spitze enthalten ist.

3. System nach Anspruch 1, bei dem die Thermoelementkomponente (306; 406) bündig mit der distalen Spitze ist.

4. System nach Anspruch 1, bei dem die Thermoelementkomponente (306; 406) über die distale Spitze hinaus vorsteht.

5. System nach einem der vorhergehenden Ansprüche, bei dem der nichtleitende Einsatz (308; 408) ein keramisches Material enthält.

6. System nach einem der vorhergehenden Ansprüche, bei dem der nichtleitende Einsatz (308; 408) weiterhin ausgestaltet ist zum elektrischen Isolieren der Thermoelementkomponente (306; 406) von dem Katheterkörper (302; 402).

7. System nach Anspruch 4, bei dem die Thermoelementkomponente (306; 406) ausgestaltet ist, über eine Mitte der distalen Spitze (304; 404) hinaus vorzustehen.

8. System nach einem der vorhergehenden Ansprüche, bei dem die distale Spitze (304; 404) einen kreisförmigen Querschnitt hat.

9. System nach Anspruch 4, bei dem die Thermoelementkomponente ausgestaltet ist, angenähert 1 mm über die distale Spitze (304; 404) hinaus vorzustehen.

10. System nach Anspruch 4, bei dem die Thermoelementkomponente ausgestaltet ist, angenähert 0,5 mm bis 1,5 mm über die distale Spitze hinaus (304; 404) vorzustehen.

11. System nach einem der vorhergehenden Ansprüche, bei dem das Thermoelement senkrecht zu einer Seitenwand der distalen Spitze (304; 404) positioniert ist.

12. System nach Anspruch 11, weiterhin aufweisend vier Thermoelemente, die um 90 Grad gegeneinander versetzt sind.

13. System nach Anspruch 1, bei dem das Thermoelement mit einem Prozessor verbunden ist, der auf der Grundlage der erfassten Temperatur eine Fluidmenge berechnet, die an der distalen Spitze (304; 404) benötigt wird, um das umgebende Gewebe zu kühlen.

14. System nach Anspruch 1 oder Anspruch 13, bei dem die Benetzungsöffnungen (322; 422) so orientiert sind, dass durch den Katheter hindurchgehendes und aus der distalen Spitze (304; 404) austretendes Fluid längsseits eines Außendurchmessers des nichtleitenden Einsatzes (308; 408) strömt.

## Revendications

1. Système de cathéter d'ablation, comprenant :
un corps de cathéter (302 ; 402) incluant une pointe distale (304 ; 404) ;
un composant de thermocouple (306 ; 406) au niveau de la pointe distale, dans lequel le composant de thermocouple est adapté de manière à détecter la température d'un fluide et/ou d'un tissu du corps ;
un insert non conducteur (308 ; 408) configuré de manière à séparer physiquement et isoler thermiquement le composant de thermocouple (306 ; 406) vis-à-vis du corps de cathéter (302 ; 402) ; et dans lequel :
le corps de cathéter inclut en outre au moins une chambre de fluide (320 ; 420), et le système comprend en outre :
une pluralité d'orifices d'irrigation (322 ; 422) à l'intérieur du corps de cathéter (302 ; 402), dans lequel les orifices de la pluralité d'orifices d'irrigation (322 ; 422) permettent à un fluide de sortir depuis l'au moins une chambre de fluide (320 ; 420) via une pluralité de canaux de circulation de fluide (324 ; 424), lesdits canaux étant séparés du composant de thermocouple par l'insert non conducteur (308 ; 408), dans lequel :
l'insert non conducteur (308 ; 408) est configuré de manière à séparer physiquement et isoler thermiquement le composant de thermocouple vis-à-vis de la pluralité d'orifices d'irrigation (322 ; 422) ; et dans lequel :
la chambre de fluide inclut une chambre de refroidissement proximale située de façon proximale par rapport à l'insert non conducteur (308 ; 408).

2. Système selon la revendication 1, dans lequel le composant de thermocouple (306 ; 406) est contenu à l'intérieur de la pointe distale.

3. Système selon la revendication 1, dans lequel le composant de thermocouple (306 ; 406) affleure la pointe distale.

4. Système selon la revendication 1, dans lequel le composant de thermocouple (306 ; 406) fait saillie depuis la pointe distale.

5. Système selon l'une quelconque des revendications précédentes, dans lequel l'insert non conducteur (308 ; 408) inclut un matériau de céramique.

6. Système selon l'une quelconque des revendications précédentes, dans lequel l'insert non conducteur (308 ; 408) est en outre adapté de manière à isoler électriquement le composant de thermocouple (306 ; 406) vis-à-vis du corps de cathéter (302 ; 402).

7. Système selon la revendication 4, dans lequel le composant de thermocouple (306 ; 406) est adapté de manière à faire saillie depuis un centre de la pointe distale (304 ; 404).

8. Système selon l'une quelconque des revendications précédentes, dans lequel la pointe distale (304 ; 404) présente une section en coupe circulaire.

9. Système selon la revendication 4, dans lequel le composant de thermocouple est adapté de manière à faire saillie sur approximativement 1 mm depuis la pointe distale (304 ; 404).

10. Système selon la revendication 4, dans lequel le composant de thermocouple est adapté de manière à faire saillie sur approximativement 0,5 mm à 1,5 mm depuis la pointe distale (304 ; 404).

11. Système selon l'une quelconque des revendications précédentes, dans lequel le composant de thermocouple est positionné perpendiculairement à une paroi latérale de la pointe distale (304 ; 404).

12. Système selon la revendication 11, comprenant en outre quatre composants de thermocouple positionnés de manière à être espacés de 90°.

13. Système selon la revendication 1, dans lequel le composant de thermocouple est connecté à un processeur adapté de manière à calculer une quantité de fluide nécessaire au niveau de la pointe distale (304 ; 404) pour refroidir un tissu avoisinant sur la base d'une température détectée.

14. Système selon la revendication 1 ou la revendication 13, dans lequel les orifices d'irrigation (322 ; 422) sont orientés de telle sorte qu'un fluide passant au travers du cathéter et sortant de la pointe distale (304 ; 404) passe le long d'un diamètre externe de l'insert non conducteur (308 ; 408).
